**Europäisches Patentamt**

**European Patent Office**

**Office européen des brevets**

(19)

(11) Veröffentlichungsnummer: **0 452 863 A2**

## (12) EUROPÄISCHE PATENTANMELDUNG

(21) Anmeldenummer: **91106011.9**

(22) Anmeldetag: **16.04.91**

(51) Int. Cl.5: **C01D 5/00**

(30) Priorität: **19.04.90 DE 4012422**

(43) Veröffentlichungstag der Anmeldung:
**23.10.91 Patentblatt 91/43**

(84) Benannte Vertragsstaaten:
**CH DE FR GB IT LI**

(71) Anmelder: **HOECHST AKTIENGESELLSCHAFT**
**Postfach 80 03 20**
**W-6230 Frankfurt am Main 80(DE)**

(72) Erfinder: **von Plessen, Helmold, Dr.**
**Kugelherrnstrasse 16**
**W-6240 Königstein/Taunus(DE)**
Erfinder: **Reichert, Karl-Michael, Dr.**
**Zeil 16**
**W-6238 Hofheim am Taunus(DE)**
Erfinder: **Metz, Erich**
**Steinwiesenweg 5**
**W-6239 Vockenhausen(DE)**

(54) **Verfahren zur Aufarbeitung von Abfallprodukten aus Abwässern der Farbstoffproduktion.**

(57) Verfahren zur Aufarbeitung von Abfallprodukten der zweistufigen Herstellung von Perylen-tetracarbonsäureanhydrid, wobei in der 1. Stufe das Perylimid gewonnen wird, welches in der 2. Stufe zum Perylen-tetracarbonsäureanhydrid verseift wird, dadurch gekennzeichnet, daß das kaliumhydroxidhaltige Abwasser der 1. Stufe und die Abfallschwefelsäure der 2. Stufe unter Gewinnung von Kaliumsulfat aufgearbeitet werden.

EP 0 452 863 A2

Die vorliegende Erfindung betrifft ein Verfahren zur Aufarbeitung von Abfallprodukten bei der zweistufigen Herstellung von Perylen-tetracarbonsäureanhydrid.

Zur Herstellung von Perylen-3,4,9,10-tetracarbonsäureanhydrid wird Naphthalin-1,8-dicarbonsäureimid in einer Schmelze aus Kaliumhydroxid und Natriumacetat bei etwa 200 bis 300 °C umgesetzt. Durch nachfolgende Oxidation mit Luftsauerstoff in wäßriger Lösung entsteht das Dikaliumsalz des Perylimids (DE-PS 276357 und EP-PS 0123256). Nach Filtration verbleibt neben dem rohen Perylimid-dikaliumsalz noch verdünnte Kaliumhydroxidlösung, die außer durch Natriumacetat hauptsächlich durch aromatische Carbonsäuren stark organisch verunreinigt ist (1. Stufe). Das Perylimid-dikaliumsalz wird anschließend mit verdünnter Mineralsäure zum Perylimid umgesetzt, das danach mit konzentrierter Schwefelsäure zu Perylentetracarbonsäureanhydrid verseift wird. (DE-PS 394794, EP-PS 0205980). Die in dieser 2. Prozeßstufe anfallende Abfallschwefelsäure ist stark organisch verunreinigt.

Die Verunreinigungen der in den beiden Prozeßstufen anfallenden Abwässer bereiten bei der Entsorgung erhebliche Probleme. So wird beispielsweise das kaliumhydroxidhaltige Abwasser zusammen mit anderen Abwässern und gegebenenfalls nach Neutralisation einer biologischen Kläranlage zugeführt. Dieses Verfahren hat den Nachteil, daß der gesamte Kaliumgehalt des Abwassers ungenutzt bleibt und als Salzbelastung des Abwassers erscheint. Ferner muß dem Abwasser zur Neutralisation gegebenenfalls noch Mineralsäure zugefügt werden, wobei sowohl der Säureverbrauch als auch der Neutralisationsprozeß zusätzliche Kosten verursachen. Zudem ergibt sich durch das Anion der hinzugefügten Mineralsäure eine zusätzliche Belastung des Abwassers.

Ein anderer Weg wird bei dem Verfahren nach DE-PS 2810878 eingeschlagen, bei dem das Alkalimetallhydroxid zurückgewonnen wird. Danach wird Abfallauge mit Eisenoxid bei Temperaturen über 800 °C zum Alkalimetallferrat umgesetzt, das dann zum Hydroxid hydrolysiert wird.

Zur Beseitigung der aus der 2. Stufe stammenden Abfallschwefelsäure wird diese im allgemeinen einem Spaltprozeß zugeführt, wobei die schwefelsauren Inhaltsstoffe bei ca. 1000 °C in der Gasphase oder in heterogener Phase reduktiv zu Schwefeldioxid und Wasser gespalten werden (Winnacker-Küchler, Chemische Technologie, 4. Aufl., Bd. 2 (1982), S. 23-25). Die über diesen aufwendigen Prozeß regenerierte Schwefelsäure ist jedoch etwa doppelt so teuer wie Schwefelsäure auf Basis von Elementarschwefel (Chem. Ind. (Düsseldorf) 40 (3), 82 - 88 (1988)).

Die vorliegende Erfindung betrifft nun ein Verfahren zur Aufarbeitung von Abfallprodukten der zweistufigen Herstellung von Perylen-tetracarbonsäureanhydrid, wobei in der 1. Stufe das Perylimid gewonnen wird, welches in der 2. Stufe zum Perylen-tetracarbonsäureanhydrid verseift wird, dadurch gekennzeichnet, daß das kaliumhydroxidhaltige Abwasser der 1. Stufe und die Abfallschwefelsäure der 2. Stufe unter Gewinnung von Kaliumsulfat aufgearbeitet werden.

Durch das erfindungsgemäße Verfahren wird es möglich, die bei der Herstellung von Perylentetracarbonsäureanhydrid anfallenden Abfälle, nämlich das kaliumhydroxidhaltige Abwasser der 1. Stufe und die Abfallschwefelsäure der 2. Stufe, in vorteilhafter Weise unter Gewinnung von Kaliumsulfat aufzuarbeiten. Das Verfahren läßt sich dabei vorzugsweise so führen, daß außer reinem Kaliumsulfat und organischem Rückstand, der verbrannt werden kann, ein Abwasser entsteht, das nach üblichen Verfahren biologisch gereinigt werden kann, so daß die genannten Nachteile des Standes der Technik entfallen.

Die Gewinnung von Kaliumsulfat aus den Abwässern der 1. und 2. Stufe der Herstellung von Perylentetracarbonsäureanhydrid erfolgt vorzugsweise dadurch, daß die Abfallsäure der 2. Stufe mit Mutterlauge des vorhergehenden Ansatzes der weiter unten erwähnten Kaliumsulfatkristallisation vermischt wird und dieses Gemisch, gegebenenfalls unter Zusatz von weiterem Kaliumsulfat, welches in diesem Fall gemäß Verfahren 2 (siehe nachfolgendes Fließschema 2, rechter Diagrammteil) durch Eindampfen der Mutterlauge der Kaliumsulfatkristallisation gewonnen wird, dem kaliumhydroxidhaltigen Abwasser der 1. Stufe zugemischt wird, worauf bei Abkühlung Kaliumsulfat kristallisiert, das abfiltriert wird. Die verbleibende Mutterlauge wird im nachfolgenden Ansatz eingesetzt.

Das erfindungsgemäße Verfahren kann sowohl unter Eindampfung der Mutterlauge der Kaliumsulfatkristallisation (Fließschema 2) als auch ohne diese Maßnahme (Fließschema 1) durchgeführt werden. Wird das erfindungsgemäße Verfahren mit einem Eindampfungsvorgang verbunden, so liegt einerseits der Vorteil in einer höheren Ausbeute an Kaliumsulfat, andererseits ist eine höhere Energiezufuhr nötig. Eine vollständige Eindampfung der ausgeschleusten $K_2SO_4$-Mutterlauge ist nicht zweckmäßig, da sich hierbei im verbleibenden Kaliumsulfat Verunreinigungen ansammeln, die bei Rückführung des so gewonnenen Kaliumsulfats in den Kristallisationsprozeß bei fortlaufendem Betrieb in steigendem Maße stören würden.

Wie weit eingedampft wird, ist von der gewünschten Reinheit und der Konzentration des Kaliumsulfats in der gebildeten Lösung abhängig. Im allgemeinen hat sich eine Eindampfung der $K_2SO_4$-Mutterlauge auf ca. 20 bis 80 Gew.-%, insbesondere 30 bis 50 Gew.-% ihrer Gewichtsmenge bewährt (siehe Beispiel 2).

Die Konzentration des eingesetzten kaliumhydroxidhaltigen Abwassers der 1. Stufe (Perylimid-Mutterl-

EP 0 452 863 A2

auge) sollte zweckmäßig 10 bis 20 Gew.-% KOH, vorzugsweise 12 bis 18 Gew.-%, betragen. Falls die Perylimid-Mutterlauge weniger als 10 Gew.-% KOH enthält, empfiehlt sich entweder eine Konzentrierung des kaliumhydroxidhaltigen Abwassers, beispielsweise durch Eindampfung vor dem Neutralisationsprozeß, oder eine weitergehende Eindampfung der ausgeschleusten $K_2SO_4$-Mutterlauge entsprechend dem Verfahren von Beispiel 2.

Die einzelnen Schritte des erfindungsgemäßen Verfahrens sind in folgender Weise gestaltet:

Wie das nachfolgende Fließschema 1, das eine bevorzugte Ausführungsform wiedergibt, zeigt, wird das Filtrat aus einem vorhergehenden Ansatz mit Abfallschwefelsäure aus der 2. Stufe der Herstellung von Perylen-tetracarbonsäureanhydrid gemischt. Das Molverhältnis von Schwefelsäure zu Kaliumsulfat beträgt vorteilhaft 1 bis 4, vorzugsweise 2 bis 3, besonders bevorzugt 2,5 bis 2,9. Die Temperatur sollte zweckmäßigerweise 10 bis 40°C, vorzugsweise 20 bis 30°C betragen. Die Abfallschwefelsäure enthält meistens 70 bis 90 Gew.-%, vorzugsweise 75 bis 85 Gew.-% und insbesondere etwa 78 bis 82 Gew.-% an $H_2SO_4$. Zu Beginn des Verfahrens wird statt des Filtrats des vorhergehenden Ansatzes eine entsprechende Menge an gesättigter Kaliumsulfatlösung vorgelegt. Durch Behandlung mit einem vorzugsweise brennbaren Adsorptionsmittel beispielsweise in Mengen von 0,1 bis 2 Gew.-%, vorzugsweise 0,2 bis 0,8 Gew.-%, bezogen auf das Gewicht der nachfolgend zugegebenen Perylimid-Mutterlauge, werden ausgeschiedene organische Substanzen abgetrennt, die als Abfall beispielsweise verbrannt werden können. Als Adsorptionsmittel kommen beispielsweise Kieselgur, Bleicherden, Bentonite, Adsorptionsharze, Aktivkoks und vorzugsweise Aktivkohle in Frage. Der so erhaltenen Lösung wird Perylimid-Mutterlauge in ungefähr stöchiometrischem Verhältnis der Reaktionspartner KOH und $H_2SO_4$ zugegeben, so daß sich ein pH-Wert von 7 bis 10, vorzugsweise von 8,5 bis 9,5 einstellt. Das Reaktionsgemisch wird dann vorzugsweise erwärmt, z.B. auf 50 bis 100°C, vorzugsweise auf 60 bis 90°C, und danach werden diesem vorzugsweise kleine Mengen, z.B. 0,2 bis 2 Gew.-%, vorzugsweise 0,5 bis 1 Gew.-%, eines Tensides, vorzugsweise eines Alkylarylpolyglykolethers oder eines langkettigen Alkyl-aminoxethylats oder -oxethylats zugegeben. Weitere geeignete Tenside sind in der DE-Patentanmeldung P 40 06 665.7 beschrieben. Anschließend kühlt man ab, z.B. auf 0 bis 20°C, vorzugsweise auf 8 bis 12°C, läßt das Kaliumsulfat auskristallisieren, filtriert es ab und wäscht es mit gesättiger Kaliumsulfatlösung. Ein der Einsatzmenge entsprechender Anteil des Filtrats wird für den folgenden Ansatz verwendet, der Rest wird als Abwasser behandelt (siehe Beispiel 1).

Soll unter Eindampfung gearbeitet werden, so wird gemäß Fließschema 2, das eine bevorzugte Ausführungsform dieser Variante darstellt, ein entsprechender Anteil der $K_2SO_4$-Mutterlauge mit Abfallschwefelsäure angesäuert, organische Substanz als brennbarer Abfall abgetrennt und die Lösung konzentriert. Nach Abkühlung filtriert man das auskristallisierte Kaliumsulfat ab und gibt es wahlweise der Perylimid-Mutterlauge oder dem Reaktionsgemisch aus $K_2SO_4$-Mutterlauge und Abfallschwefelsäure zu (Fließschema 2). Das Filtrat wird als Abwasser behandelt.

## Beispiele

1. Gemäß der Verfahrensweise des Fließschemas 1 werden 1000 g $K_2SO_4$-Mutterlauge aus einem vorhergehenden Ansatz vorgelegt. Nach Zugabe von 200 g Abfallschwefelsäure ($H_2SO_4$: 80,8 Gew.-%; C: 1,1 Gew.-%) wird mit 5 g Aktivkohle behandelt und filtriert. Das Filtrat wird zu 1000 g Perylimid-Mutterlauge (KOH: 16,5 Gew.-%; C: 1,9 Gew.-%) getropft, so daß sich bei Ende der Zugabe der pH-Wert 9 einstellt (gemessen mit gesättigter Kalomelelektrode). Nach Erwärmung des Reaktionsgemischs auf 80 bis 90°C werden 10 g eines handelsüblichen Nonylphenolpolyglykolethers zugegeben. Anschließend wird auf 10°C abgekühlt, das auskristallisierte Kaliumsulfat abfiltriert und mit 110 g gesättigter $K_2SO_4$-Lösung gewaschen. Die Waschlösung wird dem Filtrat zugegeben; die Hälfte des Gemisches wird für den nächsten Ansatz verwendet, der Rest wird als Abwasser behandelt. Nach Trocknung des Kaliumsulfats beträgt die Ausbeute 193 g = 75 % d. Th. Der Kohlenstoffgehalt des Produkts beträgt 0,017 Gew.-%.

2. Gemäß der Verfahrensweise von Fließschema 2 werden von 1900 g $K_2$-$SO_4$-Mutterlauge aus einem vorhergehenden Ansatz 900 g für die anschließende Reaktion vorgelegt, während 1000 g eingedampft werden.

Nach Zugabe von 200 g Abfallschwefelsäure ($H_2SO_4$: 81,2 Gew.-%; C: 0,7 Gew.-%) zur vorgelegten $K_2SO_4$-Mutterlauge wird mit 5 g Aktivkohle behandelt und filtriert. Das Filtrat wird zu 1000 g Perylimid-Mutterlauge (KOH 16,5 Gew.-%; C 1,9 Gew.-%) getropft, der zuvor Kaliumsulfat aus der obengenannten Eindampfung von 1000 g $K_2SO_4$-Mutterlauge zugegeben worden ist. Bei Ende der Neutralisation soll der pH-Wert des Reaktionsgemischs 9 betragen (gemessen mit gesättigter Kalomelelektrode). Nach Erwärmung auf 80 bis 90°C gibt man 5 g eines handelsüblichen Nonylphenolpolyglykolethers zu. Anschließend wird auf 10°C abgekühlt, das auskristallisierte Kaliumsulfat abfiltriert und mit 160 g gesättigter $K_2SO_4$-Lösung gewaschen. Die Waschlösung wird mit dem Filtrat vereinigt, so daß ca. 1900 g Gemisch erhalten werden. Davon werden 900 g für den folgenden Ansatz verwendet, die restlichen ca. 1000 g werden eingedampft (s. u.). Nach Trocknung des Kaliumsulfats beträgt die Ausbeute 258 g = 96 % d. Th. Der Kohlenstoffgehalt des Produkts beträgt 0,045 Gew.-%.

Eindampfung: Ca. 1000 g Filtratgemisch werden mit 28 g Abfallschwefelsäure auf den pH-Wert 3 eingestellt. Die teerartigen Ausscheidungen werden abfiltriert und verbrannt. Das Filtrat wird auf 400 g unter vermindertem Druck bei 60°C eingedampft. Das Destillat (ca. 0,6 1) wird als Abwasser behandelt. Das eingedampfte Gemisch wird auf 10°C abgekühlt und filtriert. Das Filtrat geht zum Abwasser, während das gewonnene Kaliumsulfat der Perylimid-Mutterlauge zugemischt wird.

Fließschema 1: Aufarbeitung von Perylimid-Mutterlauge durch Herstellung von Kaliumsulfat (Verfahren 1)

K$_2$SO$_4$-haltiges
Filtrat
(1000 g aus vorhergehendem Ansatz)

Abfallschwefelsäure → **Bildung von KHSO$_4$**

brennbares
Adsorptionsmittel →

**Filtration** → brennbarer Abfall

Perylimid-
Mutterlauge
(KOH-haltig) → **Neutralisation (pH 7 bis 10)**

**Erwärmung**

Tensid →

**Abkühlung Kristallisation**

Waschen mit
gesättigter
K$_2$SO$_4$-Lösung → **Filtration** → Filtrat (1000 g für folgenden Ansatz)

Kaliumsulfat
(Ausbeute)

Filtrat
(zum Abwasser)

Fließschema 2: Aufarbeitung von Perylimid-Mutterlauge durch Herstellung von Kaliumsulfat unter Eindampfen (Verfahren 2)

K$_2$SO$_4$-haltiges
Filtrat
(1900 g aus vorhergehendem Ansatz)                           Abfallschwefelsäure

                              1000 g

                     900 g

Abfallschwefelsäure            →  Bildung von   ⟵ - - - - -  Ansäuern
                   KHSO$_4$                     pH 3

brennbares
Adsorptionsmittel  →

                   Filtration      brennbarer    Filtration    brennbarer
                                   Abfall    →                  Abfall

Perylimid-
Mutterlauge     →  Neutralisation                Eindampfung    Destillat
(KOH-haltig)       (pH 7 bis 10)

                   Erwärmung                     Abkühlung
                                                 Kristallisation

Tensid          →

                   Abkühlung                     Filtration     Filtrat
                   Kristallisation                              zum
                                                                Abwasser →

                                                 K$_2$SO$_4$

Waschen mit
gesättigter
K$_2$SO$_4$-Lösung  →  Filtration

Kaliumsulfat        Filtrat
(Ausbeute)          (1900 g für
                    folgenden
                    Ansatz)

**Patentansprüche**

1. Verfahren zur Aufarbeitung von Abfallprodukten der zweistufigen Herstellung von Perylen-tetracarbonsäureanhydrid, wobei in der 1. Stufe das Perylimid gewonnen wird, welches in der 2. Stufe zum Perylen-tetracarbonsäureanhydrid verseift wird, dadurch gekennzeichnet, daß das kaliumhydroxidhaltige Abwasser der 1. Stufe und die Abfallschwefelsäure der 2. Stufe unter Gewinnung von Kaliumsulfat aufgearbeitet werden.

2. Verfahren nach Anspruch 1, dadurch gekennzeichnet, daß man die Abfallschwefelsäure aus der 2. Stufe mit Mutterlauge eines vorhergehenden Ansatzes der Kaliumsulfatkristallisation vermischt und dieses Gemisch dem kaliumhydroxidhaltigen Abwasser der 1. Stufe zumischt, Kaliumsulfat auskristallisieren läßt und dann abtrennt.

3. Verfahren nach Anspruch 1 oder 2, dadurch gekennzeichnet, daß man einen Teil der Mutterlauge eines vorhergehenden Ansatzes der Kaliumsulfatkristallisation ansäuert, filtriert und das Filtrat durch Eindampfen konzentriert und das daraus kristallisierte Kaliumsulfat wahlweise der Perylimid-Mutterlauge oder dem Reaktionsgemisch aus $K_2SO_4$-Mutterlauge und Abfallschwefelsäure zugibt.

4. Verfahren nach Anspruch 3, dadurch gekennzeichnet, daß das Filtrat auf 20 bis 80 %, insbesondere 30 bis 50 % der eingesetzten Gewichtsmenge eingedampft wird.

5. Verfahren nach einem oder mehreren der Ansprüche 1 bis 4, dadurch gekennzeichnet, daß ein brennbares Adsorptionsmittel, vorzugsweise Aktivkohle, eingesetzt wird.

6. Verfahren nach Anspruch 5, dadurch gekennzeichnet, daß 0,1 bis 2 Gew.-%, vorzugsweise 0,2 bis 0,8 Gew.-% Aktivkohle, bezogen auf das Gewicht der nachfolgend zugegebenen Perylimid-Mutterlauge, eingesetzt werden.

7. Verfahren nach einem oder mehreren der Ansprüche 1 bis 6, dadurch gekennzeichnet, daß ein Tensid, vorzugsweise ein Polyglykolether, eingesetzt wird.

8. Verfahren nach Anspruch 7, dadurch gekennzeichnet, daß das Tensid in einer Menge von 0,2 bis 2 Gew.-%, vorzugsweise 0,5 bis 1 Gew.-%, eingesetzt wird.

9. Verfahren nach Anspruch 7 oder 8, dadurch gekennzeichnet, daß das Reaktionsgemisch vor der Tensidzugabe erwärmt wird, vorzugsweise auf 60 bis 90 °C.

10. Verfahren nach einem oder mehreren der Ansprüche 7 bis 9, dadurch gekennzeichnet, daß das Reaktionsgemisch nach der Tensidzugabe auf 0 bis 20 °C, vorzugsweise auf 8 bis 12 °C, abgekühlt wird.

11. Verfahren nach einem oder mehreren der Ansprüche 1 bis 10, dadurch gekennzeichnet, daß das kaliumhydroxidhaltige Abwasser der 1. Stufe 10 bis 20 Gew.-%, vorzugsweise 12 bis 18 Gew.-% KOH, enthält.

12. Verfahren nach einem oder mehreren der Ansprüche 1 bis 11, dadurch gekennzeichnet, daß das Molverhältnis von Abfallschwefelsäure zu Kaliumsulfat aus dem Filtrat eines vorhergehenden Ansatzes 1 bis 4, vorzugsweise 2 bis 3, besonders bevorzugt 2,5 bis 2,9, beträgt.

13. Verfahren nach einem oder mehreren der Ansprüche 1 bis 12, dadurch gekennzeichnet, daß durch die Neutralisation mit dem kaliumhydroxidhaltigen Abwasser ein pH-Wert von 7 bis 10, vorzugsweise von 8,5 bis 9,5, eingestellt wird.